(19) Europäisches Patentamt
European Patent Office
Office européen des brevets

(11) **EP 0 840 887 B1**

(12) **EUROPÄISCHE PATENTSCHRIFT**

(45) Veröffentlichungstag und Bekanntmachung des Hinweises auf die Patenterteilung:
**04.02.2004 Patentblatt 2004/06**

(21) Anmeldenummer: **96945980.9**

(22) Anmeldetag: **31.10.1996**

(51) Int Cl.$^7$: **G01N 11/00**, G01N 15/00

(86) Internationale Anmeldenummer:
**PCT/DE1996/002131**

(87) Internationale Veröffentlichungsnummer:
**WO 1997/016713 (09.05.1997 Gazette 1997/20)**

(54) **VERFAHREN UND VORRICHTUNG ZUR BESTIMMUNG VON RHEOLOGISCHEN UND MECHANISCHEN STOFFKENNGRÖSSEN**

PROCESS AND DEVICE FOR DETERMINING RHEOLOGICAL AND MECHANICAL CHARACTERISTICS OF MATERIALS

PROCEDE ET DISPOSITIF POUR DETERMINER LES CARACTERISTIQUES RHEOLOGIQUES ET MECANIQUES DE MATIERES

(84) Benannte Vertragsstaaten:
**FR GB IT**

(30) Priorität: **01.11.1995 DE 19542225**

(43) Veröffentlichungstag der Anmeldung:
**13.05.1998 Patentblatt 1998/20**

(73) Patentinhaber: **L.U.M. GmbH**
**12489 Berlin (DE)**

(72) Erfinder: **LERCHE, Dietmar**
**D-12681 Berlin (DE)**

(74) Vertreter: **Wehlan, Helmut, Dr.**
**Paul-Gesche-Strasse 1**
**10315 Berlin (DE)**

(56) Entgegenhaltungen:
**EP-A- 0 392 475          EP-A- 0 447 060**
**DE-A- 4 116 313          US-A- 3 679 367**
**US-A- 4 720 465**

**Beschreibung**

Die Erfindung betrifft ein Verfahren und eine Vorrichtung zur Bestimmung von rheologischen und mechanischen Stoffkenngrößen, insbesondere disperser Medien sowie biologischer und technischer Materialien. Die Vorrichtung wirkt im Sinne eines Analyseautomaten zum gleichzeitigen Messen mehrerer Materialkenngrößen. Das Grundprinzip besteht in der Ausnutzung von variierbaren Beschleunigungskräften und der Registrierung der dadurch im Untersuchungsmaterial bewirkten Veränderungen bzw. der Verfolgung von speziell ausgebildeten Meßkörpern.

Mit ihnen wird die Bestimmung von Kenngrößen, wie Entmischungsgeschwindigkeit, Flotationsgeschwindigkeit, Partikelsorten, Partikelgrößenverteilung, Volumenkonzentration, Sedimentpackungsdichte disperser Stoffsysteme, die Bestimmung der Viskosität, Viskoelastizität und Stoffkonzentration von Fluiden und Gelen oder Dispersionsmedien sowie die Bestimmung der hydraulischen Leitfähigkeit, Elastizität, Adhäsivität, Haftfestigkeit oder Zerreißfestigkeit von Materialien möglich. Das Verfahren sowie die Vorrichtung ermöglichen es weiterhin, daß durch zyklische Meßvorgäge zeitliche Veränderungen und Reaktionsabläufe, z.B. Sol-Gel-Zustandsänderungen, dokumentiert werden.

Zur Messung von Viskositäten in fluiden Systemen, die in laufenden Prozessen eine kontinuierliche Viskositätskontrolle und -regelung erforderlich machen, sind Viskosimeter entwickelt worden, die als Rotationsviskosimeter ausgestaltet sind und die Erzeugung oder den Verbrauch von fluiden Produkten mit hoher und gleichbleibender Qualität ermöglichen sollen. In solch einer Vorrichtung wird ein koaxiales Zylindermeßsystem infolge einer zwischen Ein- und Ausströmbereich anliegenden Druckdifferenz axial durchströmt (DD 267795). Zur Charakterisierung viskoelastischer Eigenschaften von Flüssigkeiten werden z.B. Ausgangs- und Zwischenprodukte in Spinn-, Beschichtungsund anderen Formgebungsprozessen der stoffwandelnden Industrie einem Rotationsviskosimeter zugeführt, in dessen Kammer die Druckdifferenz zur freien Umgebung über der Flüssigkeitsoberfläche in der Kammer und das in einem Ringspalt auftretende Moment einer Momentenmeßeinrichtung gemessen werden (DD 267 323; DE 3826228).

Auch Rotationsrheometer, mit denen Viskositätsmessungen in unterschiedlichen Viskositätsbereichen oder Normalspannungsbereichen ermöglicht werden sollen, sind bekannt. In speziellen Ausführungsformen sind sie mit einem Kegel und einer Platte ausgestaltet, zwischen denen sich die zu messende Flüssigkeit befindet (DD 262485, DE 3815757). Auch Platte-Kegel-Viskosimeter für die serienmäßige Messung von dynamischen Viskositäten sind beschrieben worden (DE 3731317). Danach wird bei der Messung der Antriebsmotor für den Meßkegel durch das Ausgangssignal eines in einem Regelkreis eingefügten Analogrechners, der die elektrischen Signale für Drehmoment und Drehzahl kontinuierlich und gewichtet verknüpft, angesteuert.

Zur Bestimmung der Viskosität ist auch vorgeschlagen worden, eine Stahlkugel von einigen Millimetern Durchmesser durch die Einwirkung eines magnetischen Drehfeldes in Rotation zu versetzen und das in Abhängigkeit von der Viskosität des die Kugel umgebenden Mediums ausgehende Bremsmoment zu messen (DE 3442486). Die Rotation der Kugel wird mit Hilfe von Brechungsmustern sichtbar gemacht, z.B. dadurch, daß in der Rotationsachse der Kugel ein Laserstrahl auf sie trifft.

Unter den Verfahren zur Messung der Sedimentationskinetik von Bestandteilen, die sich in Flüssigkeitssuspensionen befinden, sind auch solche beschrieben worden, die unter der Einwirkung von Zentrifugalkräften vorgenommen werden.

So wird z.B. bei der Zentrifugation von Vollblut eine Lichttransmissionszunahme im Überstand gemessen, die dadurch auftritt, daß nacheinander die Erythrozyten und die Thrombozyten sedimentieren (DE 4041554). Ein motorgetriebenes Küvettenrotorsystem mit einem gegenüber dem Rotor radiär beweglichen Photometer erlaubt eine kontinuierliche Aufzeichnung der Sedimente während der ständigen Zentrifugation.

Unter dem Einfluß eines zeitlich veränderlichen Zentrifugalfeldes lassen sich auch die elastomechanischen Eigenschaften von Sedimenten aus Suspensionen und Emulsionen bestimmen (DE 4116313). Danach wird im Durchlicht-Hellfeld der Leuchtdichteverlauf von Suspensions- und Emulsionsproben auf einem Bildaufnehmer abgebildet und rechentechnisch aufbereitet. Dadurch, daß eine direkte Bestimmung der Phasengrenze entlang der Flüssigkeitssäule in Abhängigkeit von der Zentrifugalkraft vorgenommen wird, läßt sich die Elastomechanik eines zu untersuchenden Sediments bestimmen.

Mit den bekanntgewordenen Verfahren ist es nicht möglich, Viskositäts- oder Viskoelastizitätsänderungen in einem laufenden Prozeß zu bestimmen oder etwa Sol-Gel-Übergänge zu charakterisieren. Auch ist es nicht bekannt, Sedimentation und Volumenkonzentration gleichzeitig in einer Probe zu bestimmen.

Die Charakterisierung der Ausgangs- bzw. Rohstoffe bezüglich ihrer Partikelanzahl, der Partikelgröße und der Partikelgrößenverteilung ist für viele verfahrenstechnische Prozesse (z.B. Pigmente bei der Lackherstellung, Öl-Wasser- oder Wasser-Öl-Emulsionen in der Kosmetikindustrie), in der medizinischen Diagnostik (kleines und großes Blutbild) sowie in Bereichen des Umweltschutzes (Charakterisierung von technischen Abwässern) unumgänglich. Dies trifft naturgemäß besonders für die Qualitäts- und Produktkontrolle zu. Andererseits führt das zunehmende Bestreben, Herstellungsprozesse, Verfahren oder Materialeigenschaften zu optimieren, zwangsläufig auch dazu, höhere Anforderungen an die entsprechenden Meßverfahren bezüglich Meßbereich, Bedienkomfort, Genauigkeit, und Vielseitigkeit zu stellen. Insbesondere für Partikel-Flüssigkeits-Stoffsysteme (allgemein disperse Systeme), welche Partikel mit unterschiedlicher Größe, Form, Verformbarkeit und Dichte enthalten, existiert bisher noch kein praktikables und kosteneffizientes technisch realisiertes Meßverfahren. Zur Bestimmung der Teilchenzahl und/oder der Teilchengrößenverteilung existieren im wesentlichen folgende Verfahren:

1. Partikelanalyse auf konduktometrischer Basis
2. Korngrößenverteilungsanalyse durch sedimentationsfotometrische Verfahren bei Erdgravitation
3. Korngrößenanalyse im Zentrifugalfeld
4. Korngrößenanalyse und Formanalyse durch Messung des Lichtstreuverhaltens in z.B. Laserstahlmeßsystemen.

Diese Verfahren weisen unterschiedliche Einschränkungen und Unzulänglichkeiten auf. Verfahren und Vorrichtungen der ersten Gruppe bestimmen in der Regel die Partikelzahl mit hoher Zuverlässigkeit für monodisperse nichtleitende Partikel. Verteilungsfunktionen sind auf 1 bis 2 Größenordnungen beschränkt, und deformierbare Partikel werden in ihrer Größe unterbestimmt. Das Suspensionsmedium setzt eine definierte Leitfähigkeit voraus, welche durch das Einbringen der Probe nicht verändert werden darf. Verfahren und Vorrichtungen der zweiten Gruppe erlauben keine direkte Teilchenzahlbestimmung und haben für die Korngrößenbestimmung einen idealen Meßbereich von 1 bis 250 µm. Dispersionen mit geringem Dichteunterschied zwischen Suspensionsmedium und Teilchen sowie Teilchen kleiner als 1 µm benötigen eine sehr große Messzeit, und die Meßgenauigkeit wird deutlich geringer. Eine weitere prinzipielle Restriktion ist in der Forderung nach einer höheren, homogen im Suspensionsmedium verteilten Partikeldichte zu sehen. Verfahren und Vorrichtungen der dritten Gruppe sind in der Lage, Größenbereiche von 50nm bis 500µm zu erfassen. Die Hauptnachteile liegen jedoch darin, daß häufig vor der Analyse Dichtegradienten in den entsprechenden Küvetten aufzubauen sind - wodurch die Praktizierbarkeit und Anwendbarkeit der Methode stark einschränkt wird. Durch den Einsatz der 'Start-Linien' Technik und der Messung der Extinktion mittels Spezialoptik an ausgewählten Stellen in einer Küvette entstehen bei nur geringfügig verbesserten Analyseeigenschaften gleichzeitig sehr hohe Anschaffungskosten (>40.000 DM). Die automatische Messung von Teilchen mit unterschiedlichem Formfaktor bzw. mit unterschiedlicher Dichte ist bisher nicht möglich. Verfahren und Vorrichtungen der vierten Gruppe, welche im wesentlichen als 'High-End'-Geräte einzustufen sind, liefern sehr genaue Partikelzahlen und ermöglichen die Bewertung der Partikelform. Andererseits zeichnen sie sich durch sehr hohe Anschaffungskosten (> 60.000 DM) und sehr große Anforderungen bezüglich der Bedienbarkeit aus. Für den umfassenden Einsatz in labortechnischen Untersuchungen kleiner und mittlerer Unternehmen sind sie daher weniger geeignet

Die zur Zeit bekannten Verfahren und Vorrichtungen_sind bis jetzt nicht in der Lage, die dem physikalischen Prinzip des variablen Kraftfeldes (dynamisches Zentrifugalfeld) innewohnenden messtechnischen Potenzen, wie

- die ON-LINE Messungen der Teilchenparameter im gleichen Messzyklus,
- die Messungen über mehrere Größenordnungen der Teilchendichten und Teilchengrößen,
- sehr kurze Messzeiten,
- die einfache Bedienbarkeit,
- ein ergonomisches und anspruchsvolles 'Data-Handling',
- das günstige Preis-Leistungs-Verhältnis

für das Gebiet der Partikelanalyse zu nutzen.

Der Erfindung liegt die Aufgabe zugrunde, ein Verfahren und eine Vorrichtung zu entwickeln, die eine Bestimmung von rheologischen und mechanischen Stoffkenngrößen insbesondere disperser Medien ermöglichen. Die Aufgabe wurde erfindungsgemäß dadurch gelöst, daß unter Ausnutzung von variierbaren Beschleunigungskräften und der Registrierung der dadurch im Untersuchungsmaterial bewirkten Veränderungen speziell ausgebildete Meßkörper verfolgt werden und die Bewegung eines Probekörpers in einem zu untersuchenden Medium unter der Einwirkung von Beschleunigungskräften gemessen wird. Die Geschwindigkeit, mit der ein Probekörper durch ein sich veränderndes Medium wandert, stellt ein Maß für die Viskosität oder Viskoelastizität dar. Die Messung der Wanderungsgeschwindigkeit des Probekörpers erfolgt kontinuierlich auf optoelektrischem Wege. Seine Bewegung wird durch eine konstante oder durch eine variierbare Beschleunigung hervorgerufen.
Überraschenderweise hat sich herausgestellt, daß die erfindungsgemäße Lösung eine Realisierung der Multiplexizität - Ausnutzung variierbarer Beschleunigungskräfte, Registrierung der im Untersuchungsmaterial bewirken Veränderungen, Verfolgung speziell ausgebildeter Meßkörper - dadurch ermöglicht, daß wahlweise mit Probekörpern ausgestattete Meßgefäße eingesetzt und unterschiedliche Meßvorgänge schrittweise abgearbeitet werden.
Das erfindungsgemäße Verfahren zur automatischen Analyse geometrischer, mechanischer und rheologischer Parameter von Stoffsystemen und Materialien beruht darauf, daß auf das Untersuchungsgut und den/die Untersuchungparameter abgestimmte unterschiedliche Küvetten oder Meßsysteme, die auch in radial unterschiedlichen Positionen plaziert werden, auf einen horizontal oder vertikal positionierten Schlitten oder Rotor gebracht und einer vorgegebenen oder in Abhängigkeit von Prozessveriauf gesteuerten zeitvariablen Beschleunigung unterworfen werden. Die durch die Beschleunigung induzierte Veränderung der örtlichen und zeitlichen Zusammensetzung des Stoffsystems, der geometrischen Anordnung oder Position der Materialien oder die Lage entsprechender Probekörper wird durch mechanische oder elektromagnetische Wellen hochauflösend detektiert. On-line oder off-line werden aus diesen Signalen über entsprechende Algorithmen gleichzeitig mehrere Materialkenngrößen - wie Entmischungsgeschwindigkeit, Floatationsgeschwindigkeit, Viskosität, Viskoelastizität, Volumenkonzentration, Komgrößenverteilung, Partikelsorten, Elastizität, Adhäsivität, Haftfestigkeit oder Zerreißfestigkeit - sowie ihre Zeitabhängigkeit berechnet.

So wird für eine gleiche Probe im ersten Zeitintervall bei einer geringen Beschleunigung die Sedimentations-/Flotationsgeschwindigkeit von Teilchen oder Tröpfchen (z.B. Erythrozyten) durch Detektion der Phasengrenze zwischen der dispersen Phase (z.B. Blut) und dem Dispersionsmedium (z.B. Blutplasma) bestimmt und in einem anschließenden Zeitintervall bei einer höheren Beschleunigung die Probe vollständig entmischt. Die Anteile am Gesamtvolumen werden über eine Längenmessung erfasst, auf die ebenfalls zu detektierende Einfüllhöhe oder das bekannte Einfüllvolumen unter Berücksichtigung der Gefäßgeometrie bezogen und daraus die Volumenkonzentration oder ein adequates Maß (z.B. Hämatokrit) bestimmt

Erfindungsgemäß wird auf der Grundlage von theoretischen Gesetzmäßigkeiten oder experimentell erstellten Nomogrammen on line oder off line die ermittelte Entmischungsgeschwindigkeit der untersuchten Probe unter Verrechnung der für die gleiche Probe bestimmten Volumenkonzentration auf eine Standardkonzentration automatisch normiert.

Es hat sich herausgestellt, daß die Bewegung eines Probekörpers in einem zu untersuchenden Medium unter der Einwirkung von Zentrifugalkräften hervorgerufen, die zeitabhängige Position des Probekörpers durch mechanische oder elektromagnetische Wellen gemessen und aus dem Kraft-Bewegungs-Daten die Viskosität oder Viskoelastizität bestimmt werden kann. Dabei wird die Lage des Probekörpers im Probengefäß durch mechanische, elektrische oder magnetische Kräfte so fixiert, daß diese Kräfte von außen oder prozessgesteuert verändert werden können.

Die Bewegung des Probekörpers wird durch eine konstante, voreingestellte oder vom Prozeßverlauf abhängig variierbare Beschleunigung hervorgerufen. Der Probekörper kann durch ein elastisches Verbindungselement mit dem Probengefäß so verbunden sein, daß sich die auf Beschleunigungsänderungen erfolgende zeitabhängige Lageänderung des Probekörpers ermitteln läßt.

Das erfindungsgemäße Verfahren ist ferner dadurch charakterisiert, daß in das Meßgefäß eine Konstruktion - Parrallelschaltungen sind möglich - mit charakterisierter Geometrie eingebracht und mit dem Medium überschichtet oder unterschichtet wird. Durch die Beschleunigungskraft wird ein hydrostatischer Druck aufgebaut, der Volumenstrom durch das Filter über die Detektion der Veränderung der Ausgangsmenge oder der Filtratmenge gemessen und aus der hydraulischen Leitfähigkeit die Viskosität des Mediums berechnet Für eine gleiche Probe wird im ersten Zeitintervall bei einer geringen Beschleunigung die Sedimentations-/Flotationsgeschwindigkeit von Teilchen oder Tröpfchen (z.B. Erythrozyten) durch Detektion der Phasengrenze zwischen der dispersen Phase (z.B. Blut) und dem Dispersionsmedium (z.B. Blutplasma) bestimmt, die Lagefixierung des Probekörpers aufgehoben und im anschließenden Zeitintervall die Viskosität oder Viskoelastizität des Dispersionsmediums bestimmt.

Auf der Grundlage von theoretischen Gesetzmäßigkeiten oder experimentell erstellten Nomogrammen wird on line oder off line die ermittelte oder normierte Entmischungsgeschwindigkeit der untersuchten Probe unter Verrechnung der für die gleiche Probe bestimmten Dispersionsmittelviskosität auf eine Standardviskosität automatisch normiert.

Erfindungsgemäß wird eine bestimmte Menge der Probe in ein zusätzliches Probenaufnahmegefäß - das mit einem geeigneten Reagens gefüllt ist, welches die disperse Phase teilweise oder vollständig auflöst - gegeben. Die nicht aufgelösten Bestandteile werden wahlweise durch eine geeignete Beschleunigung separiert und die Menge der in Lösung gehenden Stoffe (z.B. Inhaltsstoffe von Zellen) oder der verbleibenden, nicht separierbaren Mikroteilchen (z.B. Zellorganellen) durch mechanische oder elektromagnetische Wellen oder die Bewegung von Probekörpern gemessen und unter Verwendung von Nomogrammen die Ausgangskonzentration der dispersen Phase der Untersuchungsprobe ermittelt

Im Falle von polydispersen Systemen (z.B. Blut) kommen Reagenzien zum Einsatz, die nur eine Fraktion der dispersen Phase auflösen oder verändern. Anschließend wird die Volumenkonzentration der Restphase nach Entmischung bei einer geeigneten Beschleunigung und die Konzentration der durch das Reagens aufgelösten oder veränderten Teilfraktion bzw. nicht separierbarer Mikroteilchen bestimmt

Im Falle von polydispersen Systemen wird ein bestimmtes Volumen der - falls notwendig vorverdünnten - Meßprobe in ein zusätzliches Meßgefäß gegeben und die zeitliche Veränderung während der Entmischung in der hochverdünnten Lösung in Abhängigkeit von der radialen Position registriert. Dann erfolgt eine progammtechnische Umsetzung des diesen Entmischungsvorgang beschreibenden Gleichungssystems. Aus dem Vergleich der experimentellen und der theoretischen Modellierung werdem die Gesamtvolumenkonzentration, die fraktionelle Volumenkonzentrationen und die Komgrößenverteilung ermittelt. Die Bestimmung erfolgt aus einer radial ortsaufgelösten Messung zu einem fest vorgegebenen oder zu einem in Abhängigkeit vom Prozeßverlauf ermittelten Zeitpunkt.

Im Fall von polydispersen Systemen mit Teilchen unterschiedlicher Dichte und Größe werden die Meßprobe in Meßgefäße mit Trägermedien unterschiedlicher Dichte gegeben, eine vorgegebene oder durch den Prozess gesteuerte zeitlich veränderliche Beschleunigung realisiert und zusätzlich die Dichteverteilung der dispersen Phase ermittelt.

Erfindungsgemäß können eine oder mehrere Fraktionen der dispersen Phase zur besseren Volumen- oder Stoffkonzentrationsbestimmung in geeigneter Weise markiert oder gefärbt werden.

Aus einer bestimmten scheinbaren Volumenkonzentration (Probengefäß A) und einer bestimmten Stoffkonzentration (Probengefäß B) werden unter Berücksichtigung der eingesetzten Probenvolumina die Porosität der entmischten dispersen Phase bzw. die absolute Volumenkonzentration, der Einfluß von Teilfraktionen im Falle polydisperser Systeme auf diese Parameter und aus dem Vergleich zwischen der scheinbaren und der absoluten Volumenkonzentration die Packbarkeit der dispersen Phase bzw. die Verformbarkeit der dispersen Teilchen automatisch bestimmt. Dabei wird in eines der Probengefäße eine Standarddispersion bekannter Teilchen-Teilchen-Wechselwirkung (z.B. aggregierende Erythrozyten) eingebracht, der Entmischungsverlauf in beiden Küvetten registriert und durch den Vergleich des Entmischungsverhaltens und / oder - verlaufs die Wechselwirkung der unbekannten Dispersion als relative, auf diesen Standard bezogene Größe bestimmt.

Die Erfindung ist ferner dadurch charakterisiert, daß die Beschleunigung schrittweise in Abhängigkeit von der Veränderung des Entmischungszustandes erhöht, registriert und unter wahlweiser Einbeziehung der Volumenkonzentration und der Dispersionsmediumsviskosität die Verformbarkeit der Bestandteile der dispersen Phase bzw. die Packbarkeit der entmischten dispersen Phase charakterisiert wird.

Erfindungsgemäß werden auf den Rotor geeignete Probenbehälter in radial unterschiedlicher Position plaziert, der Entmischungsverlauf in beiden Küvetten nach einer festlegbaren Zeit t1 registriert, danach der Entmischungsprozeß bei konstanter oder geänderter Rotordrehzahl fortgesetzt, der Entmischungsverlauf der in Drehachsennähe plazierten Küvette weiter verfolgt und eine für das Deformationsverhalten / Packbarkeit charakteristische Zeit t2 durch den Vergleich des Entmischungsverhaltens und / oder - verlaufs mit dem der drehachsenfernen Küvette zum Zeitpunkt t1 bestimmt In eine der beiden Küvetten wird eine Standarddispersion bekannter Deformierbarkeit / Packbarkeit eingebracht und die Deformierbarkeit / Packbarkeit der unbekannten Dispersion als relative, auf diesen Standard bezogene Größe bestimmt

Ein weiteres Merkmal der Erfindung besteht darin, die Schicht eines an einer Testplatte adhärierenden oder haftenden Materials auf Intaktheit und Vollstädigkeit der Beschichtung zu prüfen, einer variablen ansteigenden Beschleunigung auszusetzen, den Zeitpunkt erster Defekte der Beschichtung sowie der Ablöseverlauf der gesamten Schicht mittels mechanischer oder elektromagnetischer Wellen aufzuzeichnen und als Funktion der wirkenden Beschleunigungskraft darzustellen.

Es ist auch möglich, ein auf mechanische Festigkeit zu testendes Material auf der Beschleunigungsvorrichtung fest einzuspannen, an dem Material ein Gewicht zu befestigen, diesen Verbund einer Beschleunigungskraft auszusetzen, die Verformung und die Ruptur des Testmaterials aufzuzeichnen und über die Beschleunigungskraft Werkstoffparameter zu berechnen.

Erfindungsgemäß läßt sich in ein speziell gestaltetes Meßgefäß auch ein Testmaterial mit unbekannter Porengeometrie bringen, mit einem Medium bekannter Viskosität überschichten oder unterschichten, durch die Beschleunigungskraft ein hydrostatischer Druck aufbauen, der Volumenstrom durch das Filter über die Detektion der Veränderung der Ausgangsmenge bzw. der Filtratmenge oder eine Konzentrationsbestimmung messen und aus Volumenstrom und Viskosität die hydraulische Leitfähigkeit berechnen.

Die Erfindung ist ferner dadurch charakterisiert, daß die Beschleunigungsänderung zyklisch erfolgt und die Auslenkung des Probekörpers, Frequenz, Resonanzfrequenz und Frequenzverschiebung ermittelt und als Funktion der Zeit registriert werden. Dabei kann während der gesamten Messung die Temperatur registriert und in die Auswertungsschritte eingerechnet werden.

Die zu untersuchenden Medien stellen in erster Linie Suspensionen Emulsionen, Abwässer und Aufschlämmungen biologischen oder technischen Ursprungs dar. Bei den gemäß der vorliegenden Erfindung zu untersuchenden Medien handelt es sich um Flüssigkeiten, Suspensionen, Emulsionen, Schäume oder Gele, bei den zu untersuchenden Materialien in erster Linie um biologische Zellen, Gewebe, Biomaterialien und Verbundwerkstoffe geringer Haft- und Zerreißfestigkeit.

Die Erfindung beruht auch auf einer Kombination bekannter Verfahrensweisen und Vorrichtungen sowie bekannter und neuer Schritte bzw. Geräteteile. Die kombinierte Anwendung bekannter und neuer Techniken führt insofern zu einem synergistischen Effekt, als sie Messungen möglich macht - etwa die gleichzeitige Bestimmung mehrerer Stoffkenngrößen disperser Medien - die bislang nicht zu realisieren waren.

Die erfindungsgemäße Vorrichtung zur automatischen Analyse geometrischer, mechanischer und rheologischer Parameter von Stoffsystemen und Materialien besteht aus Spezialmeßgefäßen und Prüfvorrichtungen, aus einer steuerbaren Antriebseinheit zur Erzeugung der Beschleunigungskräfte, einem für die Aufnahme der Meßgefäße oder Prüfvorrichtungen konzipierten und beschleunigbaren Schlitten oder Rotor, einer oder mehrerer auf mechanischen oder elektromechanischen Wellen basierende Sensoreinrichtungen zur Erfassung der durch die Beschleunigungskräfte bewirkten Veränderungen der zeitlichen und örtlichen Zusammensetzung des Stoffsystems, der geometrischen Anordnung oder Position des Materials, der Lage von Phasengrenzen und entsprechender Probekörper, einer oder mehrerer Auswerteeinheiten zur Wandlung der Sensorprimärsignale in rechentechnisch nutzbare Signale, eine Mikrocontroller- oder Mikrorechnereinheit für die on line Speicherung und Steuerung der für den Betrieb und die Auswertung notwendigen Informationen, ein Softwarepaket zur Ermittlung der Stoffund Materialkenngrößen aus den gewandelten Meßsignalen, den Nomogrammen und den eingesetzten Beschleunigungskräften.

Die Vorrichtung ist ferner dadurch charakterisiert, daß die Spezielmeßgefäße eine unterschiedliche Geometrie haben, mit oder ohne Reagenzien gefüllt eingesetzt werden und mit oder ohne Probekörper ausgestaltet sind. Besondere Ausgestaltungsformen betreffen die Geometrie der Gefäße und der Probekörper, die verwendeten Reagenzien, die Lage der Probekörper (frei oder befestigt) sowie die Art und Weise ihrer Befestigung.

Eine Ausgestaltungsform der Vorrichtung findet sich in dem Gerät "LUMiFuge" (Beispiel 1). Das Meßprinzip dieses Gerätes besteht in der Erfassung des rotationsverursachten Entmischungsprozesses der Blutproben mittels optoelektronischen Sensors. Dabei wird während der Rotation für jeden zeitlichen Meßpunkt das Transmissionsprofil der Probe registriert.

Die Erfindung soll anhand von Ausführungsbeispielen näher erläutert werden, ohne daß sie sich in den Beispielen erschöpft.

**Ausführungsbeispiele**

Beispiel 1: Funktionsprinzip der LUMiFuge

Beispiel 2: BSG-Bestimmung

Beispiel 3: Hämatokritbestimmung

**Einführung**

In Deutschland werden pro Jahr ca. 48,3 Millionen Bestimmungen der Blutsenkungsgeschwindigkeit entsprechend der WESTERGREN-Methode durchgeführt. Diese Untersuchungen sind langwierig und eine wesentliche, für die Bewertung wichtige Einflußgröße - der Hämatokrit (Hk) - muß separat ermittelt werden. Aus klinischpraktischer Sicht ist daher die Entwicklung einer zeiteffizienten Methode zur objektiven Bestimmung sowohl der BSG, als auch gleichzeitig des Hk notwendig.

In diesem Beitrag stellen wir Ergebnisse vor, die mit einem neuartigen Analysesystem - der LUMiFuge - erzielt wurden. Dieses gerät ermöglicht es, innerhalb von zehn Minuten sowohl die BSG, als auch den Hämatokrit der Blutprobe zu bestimmen.

**Funktionsprinzip der LUMiFuge**

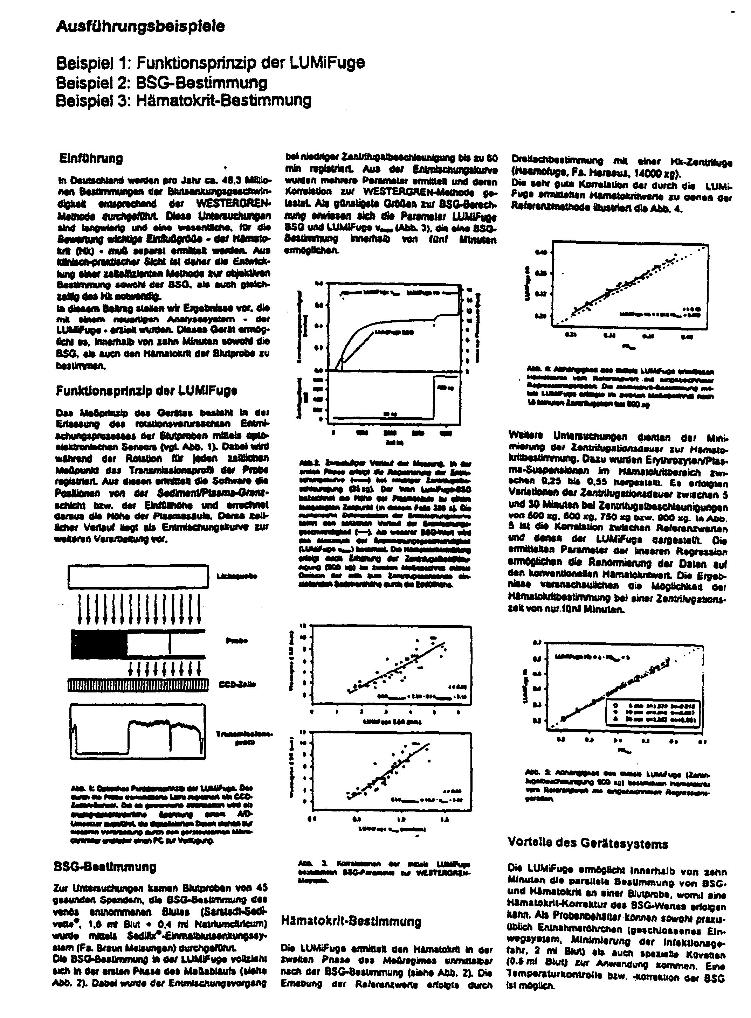

Das Meßprinzip des Gerätes besteht in der Erfassung des rotationsverursachten Entmischungsprozesses der Blutproben mittels optoelektronischen Sensors (vgl. Figur 1). Dabei wird während der Rotation für jeden zeitlichen Meßpunkt das Transmissionsprofil der Probe registriert. Aus diesen ermittelt die Software die Positionen von der Sediment/Plasma-Grenzschicht bzw. der Einfüllhöhe und errechnet daraus die Höhe der Plasmasäule. Deren zeitlicher verlauf liegt als Entmischungskurve zur weiteren Verarbeitung vor.

Figur 1: Optisches Funktionsprinzip der LUMiFuge. Das durch die Probe (1) transmittierte Licht einer Lichtquelle (2) registriert ein CCD-Zeilen-Sensor (3). Die so gewonnene Information (Transmisionsprofil 4) wird als analog-diskontinuierliche Spannung einem A/D-Umseter zugeführt, die digitalisierten Daten stehen zur weiteren Verarbeitung durch den geräteintemen Mikrocontroller und/oder einen PC zur Verfügung.

**BSG-Bestimmung**

Zur Untersuchungen kamen Blutproben von 45 gesunden Spendern, die BSG-Bestimmung des venös entnommenen Blutes (Sarstedt-Sedivette®, 1,6 ml Blut + 0,4 ml Natiumcitricum) wurde mittels Sedifix®-Einmalblutsenkungssystem (Fa. Braun Melsungen) durchgeführt.

Die BSG-Bestimmung in der LUMiFuge vollzieht sich in der ersten Phase des Meßablaufs (siehe Figur 2). Dabei wird der Entmischungsvorgang bei niedriger Zentrifugalbeschleunigung bis zu 60 min registriert. Aus der Entmischungskurve wurden mehrere Parameter ermittelt und deren Korrelation zur WESTERGREN-Methode getestet. Als günstigste Größen zur BSG-Berechnung erwiesen sich die Parameter LUMiFuge BSG und LUMiFuge $V_{max}$ (Figur 3), die eine BSG-Bestimmung innerhalb von fünf Minuten ermöglichen.

Figur 2: Zweistufiger Verlauf der Messung. In der ersten Phase erfolgt die Registrierung der Entmischungskurve (—) bei niedriger Zentrifugalbeschleunigung (25 xg) Der Wert LumiFuge-BSG (5) bezeichnet die Höhe der Plasmasäule zu einem festgelegten Zeitpunkt (in diesem Falle 288 s). Die numerische Differentiation der Entmischungskurve liefert den zeitlichen Verlauf der Entmischungsgeschwindigkeit (---). Als weiterer BSG-Wert wird das Maximum der Entmischungsgeschwindigkeit (LUMiFuge $V_{max}$, 6) bestimmt. Die Hämatokritermittlung (7) erfolgt nach Erhöhung der Zentrifugalbeschleunigung (900 xg) im zweiten Meßabschnitt mittels Division der sich zum Zentrifugationsende einstellenden Sedimenthöhe durch die Einfüllhöhe.

Figur 3: Korrelationen der mittels LUMiFuge bestimmten BSG-Parameter zur WESTERGREN-Methode.

$$ESR_{Westergren} = 2,28 \cdot ESR_{LUMiFuge} -3,16, \ r = 0,92$$

$$ESR_{Westergren} = 10,6 \cdot V_{max} - 3,92, \ r = 0,93$$

## Hämatokrit-Bestimmung

Die LUMiFuge ermittelt den Hämatokrit in der zweiten Phase des Meßregimes unmittelbar nach der BSG-Bestimmung (siehe Figur 2). Die Erhebung der Referenzwerte erfolgte durch Dreifachbestimmung mit einer Hk-Zentrifuge (Haemofuge, Fa. Heraeus, 14000 xg) Die sehr gute Korrelation der durch die LUMi-Fuge ermittelten Hämatokritwerte zu denen der Referenzmethode illustriert Figur 4.

Figur 4: Abhängigkeit des mittels LUMiFuge ermittelten Hämatokrits vom Referenzwert mit eingezeichneter Regressionsgeraden. Die Hämatokrit-Bestimmung mittels LUMiFuge erfolgte im zweiten Meßabschnitt nach 15 Minuten Zentrifugation bei 900 xg.

$$LUMiFuge \ Hk = 1,015 \ Hk_{Ref} + 0,002, \ r = O,99$$

Weitere Untersuchungen dienten der Minimierung der Zentrifugationsdauer zur Hämatokrit-Bestimmung. Dazu wurden Erythrozyten/Plasma-Suspensionen im Hämatokritbereich zwischen 0,25 bis 0,55 hergestellt. Es erfolgten Variationen der Zentrifugationsdauer zwischen 5 und 30 Minuten bei Zentrifugalbeschleunigungen von 500 xg, 600 xg, 750 xg bzw. 900 xg. In Figur 5 ist die Korrelation zwischen Referenzwerten und denen der LUMiFuge dargestellt. Die ermittelten Parameter der linearen Regression ermöglichen die Korrektur der Daten auf den konventionell bestimmten Hämatokritwert. Die Ergebnisse veranschaulichen die Möglichkeit der Hämatokritbestimmung bei einer Zentrifugationszeit von nur fünf Minuten.

Figur 5: Abhängigkeit des mittels LUMiFuge (Zentrifugalbeschleunigung 900 xg) bestimmten Hämatokrits vom Referenzwert mit eingezeichneten Regressionsgeraden.

$$LUMiFuge \ Hk = a \cdot Hk_{Ref} + b$$

$$8 - 5 \ min \ a = 1,076 \ b = -0,010$$

$$9 - 10 \ min \ a = 1,046 \ b = -0,007$$

$$10 - 20 \ min \ a = 1,002 \ b = +0,001$$

## Vorteile des Gerätesystems

Die LUMiFuge ermöglicht innerhalb von zehn Minuten die parallele Bestimmung von BSG- und Hämatokrit an einer Blutprobe, womit eine Hämatokrit-Korrektur des BSG-Wertes erfolgen kann. Als Probenbehälter können sowohl praxisüblich Entnahmeröhrchen (geschlossenes Einwegsystem, Minimierung der Infektionsgefahr, 2 ml Blut) als auch spezielle Küvetten (0,5 ml Blut) zur Anwendung kommen. Eine Temperaturkontrolle bzw. -korrektion der BSG ist möglich.

## Patentansprüche

1. Verfahren zur Bestimmung von rheologischen und mechanischen Stoffkenngrößen durch Analyse geometrischer, mechanischer und rheologischer Parameter von Medien und Materialien, **gekennzeichnet dadurch, dass** auf das Untersuchungsgut und den/die Untersuchungsparameter abgestimmte unterschiedliche Küvetten oder Meßsysteme einer zeitvariablen Beschleunigung unterworfen werden, die durch die Beschleunigung induzierte Veränderung der örtlichen und zeitlichen Zusammensetzung des Untersuchungsguts, der geometrischen Anordnung oder Position der Materialien oder die Lage entsprechender Probekörper durch mechanische oder elektromagnetische Wellen hochauflösend detektiert und aus diesen Signalen gleichzeitig mehrere Stoffkenngrößen sowie ihre Zeitabhängigkeit ermittelt werden.

2. Verfahren nach Anspruch 1, **gekennzeichnet dadurch, dass** für die gleiche Probe im ersten Zeitintervall bei einer geringen Beschleunigung die Sedimentations/Flotationsgeschwindigkeit von Teilchen oder Tröpfchen (z.B. Erythrozyten) durch Detektion der Phasengrenze zwischen der dispersen Phase (z.B. Blut) und dem Dispersionsmedium (z.B. Blutplasma) bestimmt wird und in einem anschließenden Zeitintervall bei einer höheren Beschleunigung die Probe vollständig entmischt wird, die Anteile am Gesamtvolumen über eine Längenmessung erfasst werden, auf die ebenfalls zu detektierende Einfüllhöhe oder das bekannte Einfüllvolumen unter Berücksichtigung der Gefäßgeometrie bezogen und daraus die Volumenkonzentration oder ein adäquates Maß (z.B. Hämatokrit) sowie die Packbarkeit der dispersen Phase bzw. die Verformbarkeit der dispersen Teilchen oder Tröpfchen automatisch bestimmt wird.

3. Verfahren nach Anspruch 1, **gekennzeichnet dadurch, dass** die Bewegung eines im Probegefäß durch mechanische, elektrische oder magnetische Kräfte, welche von außen oder prozessgesteuert verändert werden können, wahlweise fixiert oder durch ein elastisches Verbindungselement mit dem Probegefäß gekoppelten Probekörpers einem zu untersuchenden Medium unter der Einwirkung von Zentrifugalkräften hervorgerufen wird, die zeitabhängige Position des Probekörpers durch mechanische oder elektromagnetische Wellen gemessen und aus den Kraft-Bewegungs-Daten die Viskosität oder Viskoelastizität bestimmt werden.

4. Verfahren nach Anspruch 1, **gekennzeichnet dadurch, dass** in das Meßgefäß eine Konstruktion, Parallelschaltungen sind möglich, mit charakterisierter Geometrie eingebracht, mit dem Medium überschichtet oder unterschichtet wird, durch die Beschleunigungskraft ein hydrostatischer Druck aufgebaut, der Volumenstrom durch das Filter über die Detektion der Veränderung der Ausgangsmenge oder der Filtratmenge gemessen und aus der hydraulischen Leitfähigkeit die Viskosität des Mediums berechnet wird.

5. Verfahren nach Anspruch 1, **gekennzeichnet dadurch, dass** eine bestimmte Menge der Probe in ein zusätzliches Probenaufnahmegefäß - das mit einem geeigneten Reagens gefüllt ist, welches eine disperse Phase teilweise oder vollständig auflöst - gegeben wird, die nicht aufgelösten Bestandteile wahlweise durch eine geeignete Beschleunigung separiert werden und die Menge der in Lösung gehenden Stoffe (z.B. Inhaltsstoffe von Zellen) oder der verbleibenden, nicht separierbaren Mikroteilchen (z.B. Zellorganellen) durch mechanische oder elektromagnetische Wellen oder die Bewegung von Probekörpem gemessen und unter Verwendung von Nomogrammen die Ausgangskonzentration der dispersen Phase der Untersuchungsprobe ermittelt wird.

6. Verfahren nach Anspruch 1, **gekennzeichnet dadurch, dass** im Falle von polydispersen Systemen ein bestimmtes Volumen der - falls notwendig vorverdünnten - Meßprobe in zusätzliche Meßgefäße, welche ein Trägermedium unterschiedlicher Dichte enthalten können, gegeben wird, die zeitliche Veränderung infolge der Zentrifugalkräfte während der Entmischung in der hochverdünnten Lösung in Abhängigkeit von der radialen Position registriert wird, eine progammtechnische Umsetzung des diesen Entmischungsvorgang beschreibenden Gleichungssystems erfolgt und aus dem Vergleich der experimentellen und der theoretischen Mödellierung Gesamtvolumenkonzentration, fraktionelle Volumenkonzentrationen und die Korngrößenverteilung oder Dichteverteilung der dispersen Phase ermittelt wird.

7. Verfahren nach Anspruch 1, 2 und 5, **gekennzeichnet dadurch, dass** eine oder mehrere Fraktionen der dispersen Phase zur besseren Volumen- oder Stoffkonzentrationsbestimmung in geeigneter Weise markiert oder gefärbt werden kann.

8. Verfahren nach Anspruch 1, **gekennzeichnet dadurch, dass** die Schicht eines an einer Testplatte adhärierenden oder haftenden Materials auf Intaktheit und Vollständigkeit der Beschichtung geprüft oder ein auf mechanische Festigkeit zu testendes, mit einer Masse gekoppelten Materials, einer variablen ansteigenden Beschleunigung ausgesetzt, der Zeitpunkt erster Defekte der Beschichtung sowie der Ablöseverlauf der gesamten Schicht mittels mechanischer oder elektromagnetischer Wellen aufgezeichnet und als Funktion der wirkenden Beschleunigungskraft dargestellt wird.

9. Verfahren nach Anspruch 1, **gekennzeichnet dadurch, dass** auf den Rotor geeignete Probenbehälter in radial unterschiedlicher Position platziert werden, der Entmischungsverlauf in beiden Küvetten nach einer festlegbaren Zeit t1 registriert wird, danach der Entmischungsprozess bei konstanter oder geänderter Rotordrehzahl fortgesetzt, der Entmischungsverlauf der in Drehachsennähe platzierten Küvette weiter verfolgt und eine für das Deformationsverhalten / Packbarkeit charakteristische Zeit t2 durch den Vergleich des Entmischungsverhaltens und / oder -verlaufs mit dem der drehachsenfernen Küvette zum Zeitpunkt t1 bestimmt wird.

10. Vorrichtung zur Analyse geometrischer, mechanischer und rheologischer Parameter von Medien und Materialien, bestehend aus Spezialmeßgefäßen und Prüfvorrichtungen, aus einer steuerbaren Antriebseinheit zur Erzeugung der Beschleunigungskräfte, einem für die Meßgefäße oder Prüfvorrichtungen konzipierten Aufnahmesystem, Sensoreinrichtungen zur Erfassung der durch die Beschleunigungskräfte bewirkten Veränderungen der zeitlichen und örtlichen Zusammensetzung des Stoffsystems, der geometrischen Anordnung oder Position des Materials, der Lage von Phasengrenzen und entsprechender Probekörper, Auswerteeinheiten zur Wandlung der Sensorprimärsignale, einer Mikrocontroller- oder Mikrorechnereinheit, sowie einem Softwarepaket zur gleichzeitigen Ermittlung mehrerer Stoffkenngrößen sowie ihrer Zeitabhängigkeit.

## Claims

1. A process for determining rheological and mechanical characteristics of materials by analyzing geometric, mechanical and rheological parameters of media and materials, **characterized in that** different cells or measurement systems adapted to the test product and the test parameter(s) are subjected to a time-variable acceleration, the acceleration-induced change of the local and temporal composition of the test product, the geometric arrangement or position of the materials, or the position of respective sample bodies is detected at a high resolution by means of mechanical or electromagnetic waves, and, from these signals, various characteristics of materials as well as their time-dependence are determined.

2. The process according to claim 1, **characterized in that** the sedimentation/flotation velocity of particles or droplets (e.g. erythrocytes) is determined for the same sample in the first time interval at a slight acceleration by detecting the phase boundary between the disperse phase (e.g. blood) and the dispersion medium (e.g. blood plasma), and in a subsequent time interval, the sample is completely separated at a higher acceleration, the shares of the total volume are recorded through a length measurement, are put into relation to the filling level likewise to be detected or to the filling volume known having regard to the vessel geometry, and from this, the volume concentration or an adequate measure (e.g. haematocrit), as well as the packing capacity of the disperse phase or the deformability of the disperse particles or droplets is automatically determined.

3. The process according to claim 1, **characterized in that** the movement of a sample body within a sample vessel may be caused by mechanical, electrical or magnetic forces that can be changed from outside or in a process-controlled manner, which sample body present in a medium to be tested under the influence of centrifugal forces is optionally fixed or coupled to the sample vessel by an elastic connection element, the time-dependent position of the sample body is measured by means of mechanical or electromagnetic waves, and from the force-movement data, the viscosity or viscoelasticity is determined.

4. The process according to claim 1, **characterized in that** a construction, parallel connections are possible, having a characterized geometry is introduced into the measurement vessel, is provided with a superimposed or a lower layer of the medium, through the accelerative force, a hydrostatic pressure is built up, the volume flow through the filter is measured by detecting the change of the initial quantity or the filtrate quantity, and from the hydraulic conductivity, the viscosity of the medium is calculated.

5. The process according to claim 1, **characterized in that** a certain quantity of the sample is introduced into an additional sample receptacle that is filled with a suitable reagent, which partially or completely dissolves the disperse phase, the non-dissolved components optionally are separated by a suitable acceleration, and the quantity of the substances becoming dissolved (e.g. contents of cells) or of the remaining, non-separable microparticles (e.g. cell organelles) are measured by means of mechanical or electromagnetic waves or the movement of sample bodies, and the initial concentration of the disperse phase of the test sample is determined using nomograms.

6. The process according to claim 1, **characterized in that** in the case of polydisperse systems a certain volume of the (if necessary, pre-diluted) measurement sample is introduced into additional measurement vessels which can contain a carrier medium of a different density, and the temporal change resulting from the centrifugal forces during separation in the highly diluted solution is registered depending on the radial position, a program-controlled conversion of the equation system describing said separation process is carried out, and from the comparison of the experimental and the theoretical modeling, the total volume concentration, the fractional volume concentration and the grain size distribution or density distribution of the disperse phase are determined.

7. The process according to claims 1, 2 and 5, **characterized in that** one or more fractions of the disperse phase may be appropriately marked or colored for a better determination of volume or substance concentrations.

8. The process according to claim 1, **characterized in that** the layer of a material adhering to or sticking on a test plate is checked for integrity and completeness of the coating, or a material to be tested for mechanical strength, that is coupled to a mass, is exposed to a variable increasing acceleration, the point when defects first appear in the coating, as well as the peeling process of the entire layer is recorded by means of mechanical or electromagnetic waves, and is represented as a function of the active accelerative force.

9. The process according to claim 1, **characterized in that** suitable sample receptacles are placed on the rotor in radially differing positions, the separation process in the two cells is registered after a definable time t1, the separation process is then continued at a constant or varied rotor speed, the separation process of the cell placed near the rotational axis continues to be tracked, and a time t2 characteristic for the deformation behavior/packaging capacity is determined at a point of time t1 by comparing the separation behavior and/or separation process to that of the cell situated remote of the rotational axis.

10. A device for analyzing geometric, mechanical and rheological parameters of media and materials, comprised of special measurement vessels and test devices, a controllable driving unit for generating the accelerative forces, a reception system designed to receive the measurement vessels or test devices, sensor means for detecting the changes of the temporal and local compositions of the substance system, which changes are effected by the accelerative forces, the geometric arrangement or position of the material, the position of phase boundaries and respective sample bodies, evaluation units for converting the primary sensor signals, a microcontroller unit or microcomputer unit, and a software package for simultaneously determining various characteristics of materials and their time-dependence.

**Revendications**

1. Procédé de détermination de grandeurs caractéristiques rhéologiques et mécaniques de matériau par l'analyse de paramètres géométriques, mécaniques et rhéologiques de milieux et de matériaux, **caractérisé en ce que** les différentes cuvettes ou systèmes de mesure adaptés au produit examiné et au ou aux paramètres à examiner sont soumis à une accélération variable dans le temps, la modification induite par l'accélération de la composition en termes de localisation et de temporalité du produit à examiner, la disposition géométrique ou la position des matériaux ou l'emplacement de corps d'échantillon correspondants est détecté en haute résolution par des ondes mécaniques ou électromagnétiques et de ces signaux, plusieurs grandeurs caractéristiques de matériau ainsi que leur dépendance avec le temps sont simultanément acquises.

2. Procédé selon la revendication 1, **caractérisé en ce que,** pour le même échantillon, au cours du premier intervalle de temps, sous une faible accélération, la vitesse de sédimentation / flottation de particules ou de gouttelettes (par exemple, d'érythrocytes) est déterminée par détection de la limite de phase entre la phase dispersée (par exemple, le sang) et le milieu de dispersion (par exemple, le plasma sanguin) et que dans un intervalle de temps suivant, sous une plus forte accélération, l'échantillon est totalement dissocié, les proportions par rapport au volume total sont déterminées par une mesure des longueurs se référant à la hauteur de remplissage également à détecter ou au volume de remplissage connu, en tenant compte de la géométrie du récipient et que, à partir de cela, la concentration par volume ou une mesure adéquate (par exemple, d'hématocrite) ainsi que la capacité de tassement de la phase dispersée ou la capacité de déformation des particules ou des gouttelettes dispersées est automatiquement déterminée.

3. Procédé selon la revendication 1, **caractérisé en ce que** le mouvement d'un corps d'échantillon dans le récipient à échantillon, au choix, fixé ou couplé par un élément de liaison élastique avec le récipient à échantillon, dans un milieu à examiner, est provoqué sous l'effet de forces centrifuges, au travers de forces mécaniques, électriques ou magnétiques qui peuvent être modifiées de l'extérieur ou commandées par le processus, la position en fonction du temps du corps d'échantillon est mesurée par des ondes mécaniques ou électromagnétiques et à partir des données force - mouvement, la viscosité ou la viscoélasticité est déterminée.

4. Procédé selon la revendication 1, **caractérisé en ce que,** dans le récipient de mesure, une construction, des raccordements en parallèle sont possibles, de géométrie caractérisée est introduite, est disposée en surcouche ou en sous-couche avec le milieu, par la force d'accélération, une pression hydrostatique est créée, le débit volumique par le filtre est mesuré par la détection de la modification de la quantité initiale ou de la quantité de filtrat, et à partir de la conductivité hydraulique, la viscosité du milieu est calculée.

5. Procédé selon la revendication 1, **caractérisé en ce que,** une quantité définie de l'échantillon est apportée à un récipient à échantillon supplémentaire, qui est rempli d'un réactif adapté qui dissout totalement ou partiellement une phase dispersée, les composants non dissous sont, au choix, séparés par une accélération adaptée et la quantité de matériau passant en solution (par exemple, le matériau de contenu de cellules) ou les microparticules restantes non séparables (par exemple, des organites de cellule) est mesurée par des ondes mécaniques ou électromagnétiques ou le mouvement de corps d'échantillon est mesuré et, par l'exploitation de nomogrammes, la concentration initiale de la phase dispersée de l'échantillon examiné est déterminée.

6. Procédé selon la revendication 1, **caractérisé en ce que,** dans le cas de systèmes polydispersés, un certain volume de l'échantillon mesuré, si nécessaire, prédilué, est apporté dans des récipients de mesure supplémentaires qui peuvent contenir un milieu porteur de densités variables, la modification dans le temps en raison des forces centrifuges pendant la dissociation de mélange dans la solution très diluée en fonction de la position radiale est enregistrée, une transposition selon la technique de programme du système d'équations décrivant ce processus de dissociation de mélange s'effectue, et par la comparaison de la modélisation expérimentale et théorique, la concentration par volume totale, les concentrations par volume fractionnaire et la distribution des tailles de grain ou la distribution des densités des différentes phases sont déterminées.

**7.** Procédé selon la revendication 1, 2 et 5, **caractérisé en ce que,** une ou plusieurs fractions de la phase dispersée peuvent être marquées ou colorées de manière appropriée pour une meilleure détermination de la concentration de matériau ou par volume.

**8.** Procédé selon la revendication 1, **caractérisé en ce que** le caractère intact et complet d'une couche de matériau adhérant à ou collant sur une plaque de test est vérifié ou un matériau couplé avec une masse, dont la résistance mécanique doit être testée, est soumis à une accélération augmentante et variable, le moment auquel se crée le premier défaut dans la couche rapportée ainsi que le déroulement de du détachement de la totalité de la couche est enregistré au moyen d'ondes mécaniques et électromagnétiques et représenté en tant que fonction de la force d'accélération appliquée.

**9.** Procédé selon la revendication 1, **caractérisé en ce que** des récipients à échantillon adaptés sont placés sur le rotor, en des positions différant dans le sens radial, l'évolution de la dissociation de mélange dans les deux cuvettes est enregistrée après un temps t1 définissable, ensuite, le processus de dissociation de mélange est poursuivi à vitesse de rotation du rotor constante ou modifiée, le déroulement de la dissociation de mélange dans la cuvette placée à proximité de l'axe de rotation est encore poursuivi, et un temps t2 caractéristique du comportement à la déformation / de la capacité de tassement, est déterminé au temps t1 par comparaison du comportement et / ou de l'évolution de dissociation de mélange avec celui de la cuvette éloignée de l'axe de rotation.

**10.** Dispositif d'analyse de paramètres géométriques, mécaniques et rhéologiques de milieux et de matériaux, se composant de récipients de mesure spéciaux et de dispositifs de mesure, d'une unité d'entraînement commandable pour générer les forces d'accélération, d'un système de réception conçu pour les récipients de mesure ou les dispositifs de mesure, de dispositifs de détection pour déterminer les modifications provoquées par les forces d'accélération de la composition en termes de localisation et de temporalité du système de matériaux, de la disposition géométrique ou de la position du matériau, de l'emplacement des limites de phase et de corps d'échantillon correspondants, d'unités d'exploitation pour la transformation des signaux primaires de détecteur, d'une unité de microcontrôleur ou d'une unité de microcalculateur ainsi que d'un ensemble de logiciels pour l'acquisition simultanée de plusieurs grandeurs caractéristiques ainsi que leur dépendance du temps.

## Ausführungsbeispiele

## Beispiel 1: Funktionsprinzip der LUMiFuge
## Beispiel 2: BSG-Bestimmung
## Beispiel 3: Hämatokrit-Bestimmung

### Einführung

In Deutschland werden pro Jahr ca. 48,3 Millionen Bestimmungen der Blutsenkungsgeschwindigkeit entsprechend der WESTERGREN-Methode durchgeführt. Diese Untersuchungen sind langwierig und eine wesentliche, für die Bewertung wichtige Einflußgröße - der Hämatokrit (Hk) - muß separat ermittelt werden. Aus klinischpraktischer Sicht ist daher die Entwicklung einer zeiteffizienten Methode zur objektiven Bestimmung sowohl der BSG, als auch gleichzeitig des Hk notwendig.

In diesem Beitrag stellen wir Ergebnisse vor, die mit einem neuartigen Analysesystem - der LUMiFuge - erzielt wurden. Dieses Gerät ermöglicht es, innerhalb von zehn Minuten sowohl die BSG, als auch den Hämatokrit der Blutprobe zu bestimmen.

### Funktionsprinzip der LUMiFuge

Das Meßprinzip des Gerätes besteht in der Erfassung des rotationsverursachten Entmischungsprozesses der Blutproben mittels optoelektronischen Sensors (vgl. Abb. 1). Dabei wird während der Rotation für jeden zeitlichen Meßpunkt das Transmissionsprofil der Probe registriert. Aus diesen ermittelt die Software die Positionen von der Sediment/Plasma-Grenzschicht bzw. der Einfüllhöhe und errechnet daraus die Höhe der Plasmasäule. Deren zeitlicher Verlauf liegt als Entmischungskurve zur weiteren Verarbeitung vor.

Abb. 1: Optisches Funktionsprinzip der LUMiFuge. Das durch die Probe transmittierte Licht registriert ein CCD-Zeilen-Sensor. Das so gewonnene Meßsignal wird als analog-standardisierte Spannung erfaßt. Ein A/D-Umsetzer digitalisiert die digitalisierten Daten stehen für weitere Verarbeitung durch den gerätinternen Mikrocontroller und/oder einen PC zur Verfügung.

### BSG-Bestimmung

Zur Untersuchungen kamen Blutproben von 45 gesunden Spendern, die BSG-Bestimmung des venös entnommenen Blutes (Sarstedt-Sedivette®, 1,6 ml Blut + 0,4 ml Natriumcitricum) wurde mittels Sedifix®-Einmalblutsenkungssystem (Fa. Braun Melsungen) durchgeführt.

Die BSG-Bestimmung in der LUMiFuge vollzieht sich in der ersten Phase des Meßablaufs (siehe Abb. 2). Dabei wurde der Entmischungsvorgang

bei niedriger Zentrifugalbeschleunigung bis zu 60 min registriert. Aus der Entmischungskurve wurden mehrere Parameter ermittelt und deren Korrelation zur WESTERGREN-Methode getestet. Als günstigste Größen zur BSG-Berechnung erwiesen sich die Parameter LUMiFuge BSG und LUMiFuge v_max (Abb. 3), die eine BSG-Bestimmung innerhalb von fünf Minuten ermöglichen.

Abb. 2: Zweistufiger Verlauf der Messung. In der ersten Phase erfolgt die Registrierung der Entmischungskurve (——) bei niedriger Zentrifugalbeschleunigung (25 xg). Der Wert LUMiFuge-BSG bezeichnet die Höhe der Plasmasäule zu einem festgelegten Zeitpunkt (in diesem Fall 236 s). Die numerische Differenzierung der Entmischungskurve liefert den zeitlichen Verlauf der Entmischungsgeschwindigkeit (—·—). Als weiterer BSG-Wert wird das Maximum der Entmischungsgeschwindigkeit (LUMiFuge v_max) bestimmt. Die Hämatokritbestimmung erfolgt nach Erhöhung der Zentrifugalbeschleunigung (900 xg) im zweiten Meßabschnitt mittels Omission der sich zum Zentrifugenzentrum orientierten Sedimenthöhe durch die Einfüllhöhe.

Abb. 3: Korrelation der mittels LUMiFuge bestimmten BSG-Parameter zu WESTERGREN-Methode.

### Hämatokrit-Bestimmung

Die LUMiFuge ermittelt den Hämatokrit in der zweiten Phase des Meßregimes unmittelbar nach der BSG-Bestimmung (siehe Abb. 2). Die Erhebung der Referenzwerte erfolgte durch Dreifachbestimmung mit einer Hk-Zentrifuge (Haemofuge, Fa. Heraeus, 14000 xg).

Die sehr gute Korrelation der durch die LUMiFuge ermittelten Hämatokritwerte zu denen der Referenzmethode illustriert die Abb. 4.

Abb. 4: Abhängigkeit der mittels LUMiFuge ermittelten Hämatokrite von Referenzwerten mit eingezeichneter Regressionsgeraden. Die Hämatokrit-Bestimmung mittels LUMiFuge erfolgte im zweiten Meßabschnitt nach 15 Minuten Zentrifugation bei 900 xg.

Weitere Untersuchungen dienten der Minimierung der Zentrifugationsdauer zur Hämatokritbestimmung. Dazu wurden Erythrozyten/Plasma-Suspensionen im Hämatokritbereich zwischen 0,25 bis 0,55 hergestellt. Es erfolgten Variationen der Zentrifugationsdauer zwischen 5 und 30 Minuten bei Zentrifugalbeschleunigungen von 500 xg, 600 xg, 750 xg bzw. 900 xg. In Abb. 5 ist die Korrelation zwischen Referenzwerten und denen der LUMiFuge dargestellt. Die ermittelten Parameter der linearen Regression ermöglichen die Renormierung der Daten auf den konventionellen Hämatokritwert. Die Ergebnisse veranschaulichen die Möglichkeit der Hämatokritbestimmung bei einer Zentrifugationszeit von nur fünf Minuten.

Abb. 5: Abhängigkeit der mittels LUMiFuge (Zentrifugalbeschleunigung 900 xg) bestimmten Hämatokrits von Referenzwerten mit eingezeichneten Regressionsgeraden.

### Vorteile des Gerätesystems

Die LUMiFuge ermöglicht innerhalb von zehn Minuten die parallele Bestimmung von BSG und Hämatokrit an einer Blutprobe, womit eine Hämatokrit-Korrektur des BSG-Wertes erfolgen kann. Als Probenbehälter können sowohl praxisüblich Entnahmeröhrchen (geschlossenes Einwegsystem, Minimierung der Infektionsgefahr, 2 ml Blut) als auch spezielle Küvetten (0,5 ml Blut) zur Anwendung kommen. Eine Temperaturkontrolle bzw. -korrektur der BSG ist möglich.